# EUROPEAN PATENT SPECIFICATION

(11) **EP 4 021 607 B1**
(45) Date of publication and mention of the grant of the patent: **15.11.2023**
(21) Application number: 20756966.6
(22) Date of filing: 29.07.2020
(51) Int. Cl.: B01D 15/26, B01D 11/02, C07C 51/47, A61K 36/53, C07C 67/56, C07D 493/08

(54) **CARNOSIC ACID, CARNOSOL AND ROSMARINIC ACID ISOLATION METHOD**
VERFAHREN ZUR ISOLIERUNG VON CARNOSINSÄURE, CARNOSOL UND ROSMARINSÄURE
PROCÉDÉ D'ISOLATION D'ACIDE CARNOSIQUE, DE CARNOSOL ET D'ACIDE ROSMARINIQUE

(30) Priority: 26.08.2019 TR 201912820
(43) Date of publication of application: 06.07.2022
(73) Proprietor: Tübitak, 06100 Ankara (TR)
(72) Inventor: SAKLAR AYYILDIZ, Sena, 41470 Kocaeli (TR); PELVAN PEL TL , Ebru, 41470 Kocaeli (TR); KARADEN Z, Bülent, 41470 Kocaeli (TR)
(86) International application number: PCT/IB2020/057159
(87) International publication number: WO 2021/038340

(56) References cited:
- CN-A- 102 199 092
- CN-A- 102 432 469
- CN-A- 108 338 406
- CN-A- 108 420 849
- CN-B- 102 115 442
- CN-B- 103 319 495
- CN-B- 104 939 267

## Description

### Technical Field of the Invention

The invention relates to a method of separation by fractionalization of the antioxidant active ingredients of Rosemary plant (*Rosmarinus officinalis*), which are the diterpenes Carnosic acid and Carnosol and the phenolic Rosmarinic Acid, through column chromatography.

### Prior Art

There are various methods of extraction and purification in the literature to produce rosemary extract with high content of carnosic acid and carnosol, which are used as natural antioxidants in food and cosmetics industries. These methods allow the production of extracts containing carnosic acid, carnosol and rosmarinic acid in varying amounts and concentrations. These extracts are used in many products owing to their antioxidant properties; however, the amounts of active ingredients decrease proportionally during production processes. Thus, it would be a great advantage to be able to produce extracts in high concentrations with practical methods.

Within the scope of prior art,

The US patent No.US5256700A relates to the extraction of rosemary with an apolar solvent or mixture of solvents and the separation of carnosic acid by passing the extract through a column packed with an only carnosic acid-selective adsorbent together with a polar solvent. In the column, polyamide or polyvinylpyrrolidone is used as adsorbent and only carnosic acid is separated from the extract.

In the US patent No.US5859293A, rosemary is extracted with a water-miscible solvent where the pH of the solution is adjusted to a level between 7 and 10; and the compounds other than carnosic acid are precipitated. The pH of the solution is increased with sodium carbonate, sodium bicarbonate or ammonium hydroxide. By filtration or centrifugation, the carnosic acid is separated from the precipitated compounds. Then, carnosic acid is precipitated by adding sulphuric acid or acetic acid to the solvent containing carnosic acid and only the carnosic acid is separated from the solution either by filtration or centrifugation.

In the US patent No.US9376351B2, rosmarinic acid, carnosic acid and carnosol, which are the water and oil-soluble antioxidant compounds of the rosemary plant, are extracted using a mixture of water and ethyl alcohol. By evaporating the ethyl alcohol, an extract containing 25% carnosic acid and carnosol is obtained, and by removing the water, an extract containing 9% rosmarinic acid is obtained. The extracts are subjected to a deodorization process.

In the US patent No.US20030049332A1, rosemary is extracted by the use of a blend of tetrafluoroethane, acetone and methanol, and the process yields only an oil-soluble antioxidant.

In the US patent No.US3950266A, rosemary is extracted by the use of a solvent with a low boiling point like ethyl ether, and the solvent is evaporated with molecular distillation or vacuum. The extract is dissolved in a solvent with a high boiling point like cottonseed oil. The mixture is subjected to vapour distillation at 90-95°C to separate compounds giving odour and aroma. The deodorized extract is then cooled down under vacuum at room temperature; and the antioxidant is obtained as dissolved in oil.

In the international patent application No.WO2000049115A2, the plants from *Labiatae* family are extracted at 0-100°C with an organic solvent, and then, the solvent is evaporated. The concentrated solution is saturated with inert nitrogen gas at 0.1-0.5 bar in adsorption column and transferred to atmospheric pressure. The concentrated solution is re-extracted with water vapor, and the extract is filtered and separated from ethyl alcohol, water and essential oil. So, compounds (polyphenols) with antioxidant properties are obtained.

In the Chinese patent No.CN108070453A, the deoiled rosemary is extracted with ethanol-water mixture and passed through a ceramic membrane. The aqueous and solid parts are separated by centrifugation, and the solid part is extracted again with ethanol-water mixture. With the evaporation of ethyl alcohol, the carnosic acid in the aqueous part is obtained by drying. The aqueous part separated by centrifugation is dried to obtain rosmarinic acid. Filtration and centrifugation steps are repeated at least for 3 times. As a result, 5.06% rosmarinic acid and 30.26% carnosic acid are obtained.

In the Chinese patent No.CN102432469A, rosemary is extracted with 60% ethanol solution, subjected to filtration and ascorbic acid addition steps and passed through a ceramic membrane. It is passed through LSA-10 column by the use of petroleum ether and ethyl acetate. After the concentration and drying steps, rosmarinic acid and ursolic acid are separated.

In the doctoral dissertation on "Obtention and characterization of rosemary and ash tree seed extracts and study of their preventive effects on metabolic disorders" (by Sixto Alvin IBARRA ROBER, 2011), rosemary is extracted with acetone, and the solid and liquid phases are separated by centrifugation. According to the method specified in the US patent No.US5859293, base and then acid are added to the liquid phase; and carnosic acid is precipitated and separated by drying process. An extract containing 20% carnosic acid is obtained. Rosemary is extracted with water and acid is added to the extract. Rosmarinic acid is separated by fractionating in a column containing Styrene-diviniyl-benzene XAD-16 and an extract with 20% rosmarinic acid is obtained.

### Technical Problem To Be Solved By the Invention

When it is desired to reach the values of carnosic acid and carnosol recommended by EFSA for rosemary extract to have antioxidant properties, problems are encountered in the product to which the extract is added, like turbidity and rosemary aroma dominance. In order to solve such problems, the carnosic acid and carnosol concentrations obtained from rosemary need to be increased. In this way, the amount of rosemary extract to be added to a product will be reduced, on the other hand, the amounts of carnosic acid and carnosol will reach the values recommended by EFSA. Owing to this invention, the amounts of EFSA-recommended carnosic acid and carnosol -which were in a range between 3 and 26% (30mg/g-260mg/g) in previously used extraction methods- are increased up to 42 to 48%. The final product does not have a dominant rosemary odour. Therefore, it falls into deodorized product group. The efficiency is increased owing to the process conditions in the column chromatography method used in the invention (packing material, mobile phase flow program) and the conditions of sample preparation before feeding into the column. In addition, unlike other methods in the literature, surprisingly, carnosic acid, carnosol and rosmarinic acid are produced at once using the same column and the same mobile phase at a higher purity.

Also, ethanol, which is used as a solvent in the extraction of carnosic acid and carnosol from rosemary and in their fractionated separation by column chromatography, is a harmless solvent and environmentally friendly chemical used in foods.

Thanks to the invention, in the fractionating process of carnosic acid and carnosol in column chromatography system, an extract containing rosmarinic acid is obtained as a by-product with pretty high antioxidant properties. The products obtained are of deodorized nature. Rosmarinic acid is an antioxidant that dissolves in water phase and is used in water-based products, while carnosic acid and carnosol are antioxidants that dissolve in oil phase and are generally used in oil-based products.

### Descriptions of the Figures

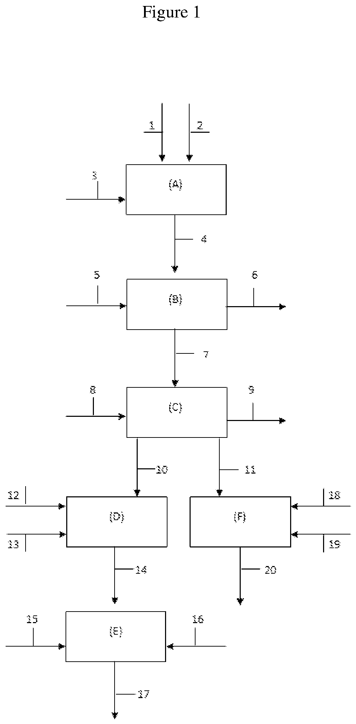

The flow diagram of the purification process designed in line with the purpose of this invention is shown in the attached Figure 1.

### Figure 1. Carnosic acid, carnosol and rosmarinic acid isolation process

### Descriptions of the References in the Figures

(A) De-oiled rosemary extraction step
   1. De-oiled rosemary
   2. 50% ethanol:water solution
   3. Heating
   4. Solid-liquid extract
(B) Filtration step
   5. Vacuum
   6. Insoluble solid matter
   7. Liquid extract
(C) Step of passing through a macro-porous adsorbent-containing column
   8. Gradient mobile phase consisting of water and/or ethanol
   9. Waste
   10. Eluent containing rosmarinic acid (Liquid extract containing rosmarinic acid)
   11. Eluent containing carnosic acid and carnosol (Liquid extract containing carnosic acid and carnosol)
(D)Step of Evaporation-1
   12. Temperature
   13. Vacuum
   14. Liquid extract rich in rosmarinic acid
(E) Freeze drying step
   15. Vacuum
   16. Cooling
   17. Rosmarinic acid extract
(F) Step of Evaporation-2
   18. Temperature
   19. Vacuum
   20. Carnosic acid+carnosol extract

### Explanation of the Invention

The invention aims to produce rosemary extract rich in carnosic acid and carnosol which are used as natural antioxidants in food and cosmetic sectors. To this end, the liquid extract obtained from rosemary plant with ethanol-water mixture is fractionated by column chromatography method using a suitable packing material and mobile phase flow program; and a deodorized extract is obtained containing 42-48% carnosic acid and carnosol. In addition to the carnosic acid and carnosol extract, an extract containing rosmarinic acid is also obtained in the column chromatography system. In this method, by making use of the suitable solution (ethanol-water) and solvent ratio (50%) for the most efficient extraction of the active ingredients from rosemary and taking advantage of the active ingredients' differences of solubility in water and ethanol and their adsorption affinities against the packing material, it is made possible to produce an extract containing 42-48% carnosic acid and carnosol and another product containing 7-8% rosmarinic acid with high antioxidant activity, at once with a more practical method compared to other methods. The liquid extracts obtained from column chromatography are dried and powdered, in rotary evaporator or lyophilizer, depending on their ethanol and water content.

Process flow diagram of the developed method is shown in Figure 1.
1- De-oiled rosemary (1) is extracted with 50% ethanol:water (v:v) solution (2). Extraction method is ultrasonic water bath at 60°C for 20 min (3).
2- The extract (4) is separated from solid matter (6) by vacuum filtration (5). The filtered liquid extract is filtered for a second time using a fine filter with 1-5 micrometer pore diameter (B).
3- The liquid extract obtained (7) is now ready for feeding into the column (C) containing a macro-porous adsorbent. Before feeding the liquid extract (7) into the column packed with styrene divinylbenzene adsorbent and water, deionized water (8) as mobile phase is passed through the column and the eluent from the column goes to waste (9).
4- The liquid extract (7) is fed into the conditioned column. Within this time period, the eluent observed at 284nm wavelength corresponding to peak-1 zone in chromatogram goes to waste (9).
5- When the feeding of the liquid extract (7) is completed, deionized water (8) as mobile phase is passed through the column and this part goes to waste (9).
6- Then, 50% ethanol-water solution (8) is passed through the column as mobile phase; meanwhile the eluent corresponding to the peak-2 zone (liquid extract containing rosmarinic acid) (10) is collected for evaporation.
7- In the next step, 99% ethanol (8) is passed through the column as mobile phase, and in the meantime, the eluent corresponding to the peak-3 zone (liquid extract containing carnosic acid and carnosol) (11) is collected for evaporation.
8- The column is washed away and cleaned by passing ethanol (8) as mobile phase so that it becomes ready for the next run. Then, deionized water (8) is passed as mobile phase and the column becomes ready for the next run (C).
9- The eluent (10) corresponding to the peak-2 zone in the column chromatography contains 50% ethanol-water, while the eluent (11) corresponding to the peak-3 zone contains only ethanol. The eluent corresponding to the peak-2 zone is subjected to vacuum (13) in rotary evaporator at 40°C (12) until the ethanol evaporates completely (D). The remaining aqueous part (14) is freeze dried at lyophilizer (15), dried (E) under vacuum (16), and rosmarinic acid extract (17) is obtained. Since the eluent (11) corresponding to the peak-3 zone is composed of ethanol only, it is subjected to vacuum (19) in rotary evaporator at 40°C (18) until it dries completely. As a result of this process (F), an extract of carnosic acid and carnosol (20) is obtained in powder form.

### Example:

200 grams of deoiled rosemary is extracted with 2 liters of (50% ethanol:water, v:v) solution. Extraction method is ultrasonic water bath at 60°C for 20 min. The extract is separated from solid matter by vacuum filtration. The filtered liquid extract is filtered for a second time using a fine filter with 1-5 micrometer pore diameter.

The liquid extract obtained is now ready for feeding into the column containing a macro-porous adsorbent. Before feeding the liquid extract into the column packed with styrene divinylbenzene Diaion HP20 adsorbent and water; deionized water as mobile phase in the amount of 0.2 times of the column volume is passed through the column and the eluent from the column goes to waste. The conditioned column is fed with liquid extract in the amount of 1.5 times of the column volume. Within this time period, the eluent observed at 284nm wavelength corresponding to peak-1 zone in chromatogram goes to waste. When the feeding of the liquid extract is completed, deionized water as mobile phase in the amount of the column volume is passed through the column and this part goes to waste. Then, 50% ethanol-water solution as mobile phase in the amount of 2 times of the column volume is passed through the column; and the eluent corresponding to the peak-2 zone (liquid extract containing rosmarinic acid) is collected for evaporation. In the next step, 99% ethanol as mobile phase in the amount of 2 times of the column volume is passed through the column, meanwhile, the eluent corresponding to the peak-3 zone (liquid extract containing carnosic acid and carnosol) is separately collected for evaporation. The column is washed away and cleaned by passing ethanol as mobile phase in the amount of the column volume, so that it becomes ready for the next run. Then, deionized water is passed as mobile phase in the amount of the column volume and the column becomes ready for the next run.

The eluent corresponding to the peak-2 zone in the column chromatography contains 50% ethanol-water, while the eluent corresponding to the peak-3 zone contains only ethanol. The eluent corresponding to the peak-2 zone is subjected to vacuum in rotary evaporator at 40°C until the ethanol in it evaporates completely. The remaining aqueous part is freeze dried at lyophilizer, dried under vacuum, and rosmarinic acid extract is obtained. Since the eluent corresponding to the peak-3 zone is composed of ethanol only, it is subjected to vacuum in rotary evaporator at 40°C until it dries completely. As a result of this process, an extract of carnosic acid and carnosol is obtained in powder form.

Analysis results on total phenolic compounds and antioxidant activity of carnosic acid, carnosol and rosmarinic acid extracts in dried powder form are given in Table 1 below.

**Table-1. Active ingredients and antioxidant activities of extracts**

| | **Amount of active ingredient, mg/100 mg** | **Total phenolic compounds, mg GAE/100mg** | **DPPH, IC₅₀ value, mg extract/ml** | **ABTS, mg TE/100 mg** |
|---|---|---|---|---|
| **Extract containing carnosic acid and carnosol** | 44.09 | 22.38 | 0.19 | 53.23 |
| **Extract containing rosmarinic acid** | 7.42 | 29.00 | 0.21 | 50.65 |

**Active ingredient amounts:** For the extract containing carnosic acid and carnosol, the amount is determined with the sum of carnosic acid and carnosol content, whereas, for the extract containing rosmarinic acid, the amount is determined with the rosmarinic acid content per 100 mg extract.

**Total phenolic compounds:** Results are given in mg gallic acid equivalent/100 mg extract.

**DPPH:** (2,2-diphenyl-1-picrylhydrazyl) radical scavenging capacity

Results are given in IC₅₀ (sample amount required to halve initial DPPH concentration in mg extract/ml solution).

**ABTS:** Trolox-equivalent (6-Hydroxy-2,5,7,8-tetramethylchromane-2-carboxylic acid) antioxidant capacity (TEAC-ABTS)

Results are given in mg Trolox-equivalent/100 mg extract.

This method makes it possible to produce deodorized extracts rich in carnosic acid and carnosol, by using plant species such as rosemary (*Rosmarinus officinalis*) and sage (*Salvia fruticosa*) from the *Labiatae* (Lamiaceae) family.

It is possible to use both deoiled rosemary and oil-containing rosemary, however, in case of oil-containing rosemary use, efficiency may decrease.

For the extraction of active ingredients, the rosemary: solvent (solid:liquid) ratio can be in the range from 1:1 to 1:200.

The solvent to be used for the extraction of active ingredients can be ethanol as well as other polar and apolar solvents like methanol, acetone and hexane. The ratio of the solvent used for the extraction of active ingredients can range from 5% to 100%.

During the feeding of the extract into the column, the ratio of extract:column volume can range from 0.01 to 5.

During fractionating by column chromatography method, it is possible to use a column chromatography system or apparatus.

As the column packing material, it is possible to use styrene divinylbenzene Diaion HP20, as well as macro-porous polyamide, polyacrylate, polymethacrylate and polyvinylpyrrolidone adsorbents.

During fractionating by column chromatography, as mobile phase, it is possible to use ethanol:water, as well as polar and apolar solvents like methanol, acetone and hexane.

In the mobile phase flow program, it is possible to start with 100% water and switch to 50% ethanol:water flow and finally to 100% ethanol flow; or instead of a stepwise transition, it is possible to switch directly from 100% water to 100% ethanol.

The obtained liquid extracts could be dried in rotary evaporator or lyophilizer, as well as in a vacuum dryer, vacuum oven or spray dryer.

If desired, the active ingredient amounts of the extracts can be increased by using a second column.

### Industrial Application of the Invention

The extracts containing carnosic acid, carnosol and rosmarinic acid can be used in food, pharmaceutical and cosmetic products both directly and by encapsulation.

## Claims

1. A method for carnosic acid, carnosol and rosmarinic acid isolation, comprising the sequential steps of
1) Extraction of deoiled rosemary (A) using ultrasonic water bath at 60°C for 20 min with a 50% ethanol:water (v:v) solution,
2) Filtration (B) of the extract of step (A) by vacuum filtration and the filtered liquid extract is filtered for a second time using a fine filter with 1-5 micrometer pore diameter,
3) Passing deionized water as a mobile phase through a macro-porous adsorbent-containing column (C) and the eluent from the column goes to waste,
4) Feeding the filtered liquid extract into the conditioned column in an amount of 0.01 to 5 times of the column volume, and observing the eluent which goes to waste at 284nm wavelength, then passing deionized water as mobile phase through the column and this part goes to waste,
5) Passing a 50% ethanol-water solution through the column as mobile phase,
6) Collection of eluent corresponding to liquid extract containing rosmarinic acid,
7) Evaporation-1 (D) of ethanol in the eluate of step 6, containing rosmarinic acid, either under vacuum in rotary evaporator, lyophilizer, vacuum dryer, vacuum oven or spray dryer,
8) Freeze drying (lyophilizing) (E) of the remaining aqueous part and obtainment of deodorized rosmarinic acid,
9) Passing a 99% ethanol through the column as mobile phase,
10) Collection of the eluent of step 9, liquid extract containing carnosic acid and carnosol,
11) Evaporation-2 (F) of ethanol in the eluate of step 10, containing carnosic acid and carnosol either under vacuum in rotary evaporator at 40 °C, until it dries completely and obtainment of deodorized carnosic acid and carnosol,

2. An isolation method according to Claim 1, **characterized in that**, the column packing material, is selected from the materials; styrene divinylbenzene, macro-porous polyamide, polyacrylate, polymethacrylate and polyvinylpyrolidone adsorbents.

3. An isolation method according to Claim 2, wherein the column packing material is styrene divinylbenzene adsorbent.

4. An isolation method according to Claim 1, **characterized in that**, during the feeding of the extract into the column, the ratio of extract: column volume is preferably 1.5.

## Patentansprüche

1. Verfahren zur Isolierung von Carnosinsäure, Carnosol und Rosmarinsäure, umfassend die folgenden aufeinanderfolgenden Schritte:
1) Extraktion von entöltem Rosmarin (A) unter Verwendung eines Ultraschall-Wasserbades bei 60°C für 20 Minuten mit einer 50%igen Ethanol-Wasser-Lösung (v:v),
2) Filtration (B) des Extrakts aus Schritt (A) durch Vakuumfiltration, wobei der filtrierte Flüssigextrakt ein zweites Mal unter Verwendung eines Feinfilters mit 1-5 Mikrometer Porendurchmesser filtriert wird,
3) Durchleiten von entionisiertem Wasser als mobile Phase durch eine makroporöse, ein Adsorptionsmittel enthaltende Säule (C), wobei das Elutionsmittel aus der Säule verloren geht,
4) Einspeisen des gefilterten Flüssigextrakts in die konditionierte Säule in einer Menge, die dem 0,01- bis 5-fachen des Säulenvolumens entspricht, und Beobachten des Elutionsmittels, das bei einer Wellenlänge von 284 nm verloren geht, dann Durchleiten von entionisiertem Wasser als mobile Phase durch die Säule, wobei dieser Teil verloren geht,
5) Durchleiten einer 50%igen Ethanol-Wasser-Lösung als mobile Phase durch die Säule,
6) Auffangen des Elutionsmittels, das dem Flüssigextrakt mit Rosmarinsäure entspricht,
7) Eindampfen-1 (D) des Ethanols im Eluat aus Schritt 6, das Rosmarinsäure enthält, entweder unter Vakuum im Rotationsverdampfer, Gefriertrockner, Vakuumtrockner, Vakuumofen oder Sprühtrockner,
8) Gefriertrocknung (Lyophilisierung) (E) des verbleibenden wässrigen Teils und Gewinnung von desodorierter Rosmarinsäure,
9) Durchleiten eines 99%igen Ethanols als mobile Phase durch die Säule,
10) Auffangen des Elutionsmittels aus Schritt 9, Flüssigextrakt mit Carnosinsäure und Carnosol,
11) Eindampfen-2 (F) des Ethanols im Eluat aus Schritt 10, das Carnosinsäure und Carnosol enthält, entweder unter Vakuum im Rotationsverdampfer bei 40°C bis zur vollständigen Trocknung und Gewinnung von desodorierter Carnosinsäure und Carnosol,

2. Isolierungsverfahren nach Anspruch 1, **dadurch gekennzeichnet, dass** das Säulenfüllmaterial aus Materialien wie Styrol-Divinylbenzol, makroporösem Polyamid, Polyacrylat, Polymethacrylat und Polyvinylpyrolidon-Adsorptionsmitteln ausgewählt ist.

3. Isolierungsverfahren nach Anspruch 2, wobei das Säulenfüllmaterial ein Styrol-Divinylbenzol-Adsorptionsmittel ist.

4. Isolationsverfahren nach Anspruch 1, **dadurch gekennzeichnet, dass** während der Zuführung des Extrakts in die Säule das Verhältnis von Extrakt zu Säulenvolumen vorzugsweise 1,5 beträgt.

## Revendications

1. Méthode d'isolation de l'acide carnosique, du carnosol et de l'acide romarinique, comprenant les étapes séquentielles suivantes :
1) Extraire de romarin déshuilé (A) à l'aide d'un bain-marie ultrasonique à 60°C pendant 20 minutes avec une solution éthanol:eau à 50% (v:v),
2) Filtrer (B) de l'extrait de l'étape (A) par filtration sous vide et l'extrait liquide filtré est filtré une seconde fois à l'aide d'un filtre fin d'un diamètre de pore de 1-5 micromètres,
3) Faire passer de l'eau désionisée comme une phase mobile à travers une colonne contenant un adsorbant macroporeux (C) et l'éluant de la colonne parvient au déchet,
4) Alimenter l'extrait liquide filtré dans la colonne conditionnée dans une quantité de 0,01 à 5 fois le volume de la colonne et observer l'éluant qui parvient au déchet à une longueur d'onde de 284nm, puis faire passer de l'eau désionisée comme une phase mobile à travers la colonne et cette partie parvient au déchet,
5) Faire passer d'une solution éthanol-eau à 50% à travers la colonne comme phase mobile,
6) Collecter de l'éluant correspondant à l'extrait liquide contenant l'acide romarinique,
7) Évaporer-1 (D) de l'éthanol dans l'éluat de l'étape 6, contenant l'acide romarinique, soit sous vide dans un évaporateur rotatif, un lyophilisateur, un séchoir à vide, une étuve à vide ou un séchoir par atomisation,
8) Sécher à froid (lyophiliser) (E) de la partie aqueuse restante et obtenir de l'acide romarinique désodorisé,
9) Faire passer d'un éthanol à 99% à travers la colonne comme phase mobile,
10) Collecter l'éluant de l'étape 9, l'extrait liquide contenant de l'acide carnosique et du carnosol,
11) Évaporer-2 (F) de l'éthanol dans l'éluat de l'étape 10, contenant l'acide carnosique et le carnosol, soit sous vide dans un évaporateur rotatif à 40°C, jusqu'à séchage complet et obtenir de l'acide carnosique et du carnosol désodorisés,

2. Méthode d'isolation selon la Revendication 1, **caractérisée par le fait que** le matériau d'emballage de la colonne est choisi parmi les matériaux tels que styrène divinylbenzène, polyamide macroporeux, polyacrylate, polyméthacrylate et adsorbants de polyvinylpyrolidone.

3. Méthode d'isolation selon la Revendication 2, dans laquelle le matériau d'emballage de la colonne est un adsorbant de styrène divinylbenzène.

4. Méthode d'isolation selon la Revendication 1, **caractérisée par le fait que**, pendant l'alimentation de l'extrait dans la colonne, le rapport extrait:volume de la colonne est de préférence de 1,5.
